# FASCICULE DE BREVET EUROPEEN

(11) **EP 3 756 715 B1**
(45) Date de publication et mention de la délivrance du brevet: **03.01.2024**
(21) Numéro de dépôt: 20180489.5
(22) Date de dépôt: 17.06.2020
(51) Int. Cl.: A61M 16/00, A61M 16/20, F17C 1/00

(54) **SYSTÈME DE DISTRIBUTION DE GAZ THÉRAPEUTIQUE COMPRENANT UNE SOUPAPE DE SÉCURITÉ**
SYSTEM ZUR THERAPEUTISCHEN GASABGABE, DAS MIT EINEM SICHERHEITSVENTIL AUSGESTATTET IST
SYSTEM FOR DISPENSING THERAPEUTIC GAS COMPRISING A SAFETY VALVE

(30) Priorité: 25.06.2019 FR 1906847
(43) Date de publication de la demande: 30.12.2020
(73) Titulaire: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Inventeur: BOULANGER, Thierry, Newark, DELAWARE 19702 (US)
(74) Mandataire: Air Liquide

(56) Documents cités:
- WO-A2-2012/164240
- GB-A- 2 486 018
- US-A1- 2003 189 492
- US-A1- 2008 302 363
- US-A1- 2014 152 468

## Description

La présente invention concerne un système de distribution de gaz thérapeutique à un patient, en particulier d'oxygène, comprenant un récipient, en particulier une bouteille de gaz, dont la sécurité a été améliorée, lequel est utilisable dans des situations où la délivrance d'un gaz thérapeutique est requise.

L'oxygène a de nombreuses applications dans le domaine médical, notamment pour traiter des patients (i.e. pédiatriques, enfants, adolescents, adultes, personnes âgées...) en situation hypoxémique souffrant de maladies ou déficiences respiratoires ou pulmonaires, par exemple des patients atteints de SDRA, de BPCO... ou encore en récupération post-opératoire.

Dans le cadre d'applications requérant une mobilité, par exemple lors de transferts intra-hospitaliers d'un patient (i.e. le patient est déplacé de sa chambre vers une salle d'IRM par exemple) ou bien dans les véhicules d'urgence (e.g. trajet entre un lieu d'intervention et un hôpital), l'oxygène administré au patient provient d'un récipient sous pression, typiquement une bouteille de gaz, et est délivré à pression constante, par exemple 5 bar abs, et à débit régulé au moyen d'un dispositif régulateur de débit. Le flux d'oxygène est ensuite convoyé jusqu'au patient via un conduit de gaz, en général un tuyau flexible, alimentant une interface respiratoire, tel qu'un masque respiratoire, des canules nasales ou analogue.

Sachant que la quantité de gaz dans un récipient de gaz, telle une bouteille de gaz, est limitée, le personnel soignant, i.e. infirmières, médecins ou analogue, doit s'assurer que la quantité d'oxygène restant dans le récipient est suffisante pour assurer les besoins du transfert, c'est-à-dire connaître son autonomie en gaz afin de pouvoir estimer la durée pendant laquelle le patient va pouvoir recevoir de l'oxygène dans le cadre de son traitement en fonction du débit d'oxygène qui lui est administré.

L'autonomie en gaz peut être obtenue par lecture soit d'une valeur de pression s'affichant sur un manomètre mécanique équipant le récipient, soit d'une valeur d'autonomie, par exemple une durée estimée (exprimée en heures et minutes), s'affichant sur un manomètre électronique, tel que décrit par WO-A-2005/093377, US- A-2008/01 50739 ou WO-A-2012/164240. US2008302363A1 divulgue un système de distribution de gaz thérapeutique comprenant un conduit de dérivation raccordé fluidiquement à un conduit de fourniture de gaz, le conduit de dérivation comprenant un capteur de pression de dérivation agencé de manière à déterminer la pression dans le conduit de dérivation et configuré pour transmettre au moins un signal de pression de dérivation à une unité de traitement de signal.

En affichant l'autonomie résiduelle de la bouteille sous forme de durée, les manomètres électroniques facilitent le travail de l'utilisateur et garantissent un premier niveau de sécurité pendant les transferts, en assurant une quantité suffisante de gaz.

Cependant, cette valeur d'autonomie affichée est relative à ce qui sort de la bouteille mais n'informe pas l'utilisateur de ce que le patient reçoit réellement, ce qui peut mener à des situations dangereuses où le patient ne reçoit plus ou plus assez d'oxygène, par exemple :
- pendant un transfert en véhicule d'urgence vers l'hôpital le plus proche, le patient ou l'utilisateur peut effectuer un mouvement inapproprié induisant une contrainte mécanique sur le conduit de gaz, lequel se déconnecte alors de la bouteille de gaz. Dans ce cas, le patient ne reçoit plus d'oxygène.
- pendant un transfert intra-hospitalier, une torsion sur le conduit de gaz peut le boucher au moins partiellement, ce qui diminue alors le débit d'oxygène administré au patient. Dans des cas extrêmes, l'occlusion peut être totale, de façon transitoire, par exemple si le lit ou un autre matériel roule ou est posé sur le conduit de gaz, et il s'ensuit alors une augmentation de pression dans le conduit pouvant induire sa déconnection de la bouteille de gaz. Dans ce cas, le patient ne reçoit pas assez, ou dans les cas extrêmes, plus d'oxygène.

Un problème à résoudre est dès lors de proposer un système de distribution de gaz à un patient comprenant un récipient, en particulier une bouteille de gaz, dont la sécurité a été améliorée de manière à limiter les problèmes et inconvénients susmentionnés, c'est-à-dire en limitant la possibilité de déconnection du conduit de gaz en réponse à son obstruction totale, mais aussi d'alerter l'utilisateur, e.g. personnel soignant, d'un défaut de fourniture d'oxygène au patient résultant d'une déconnection ou d'une obstruction du conduit de gaz.

L'invention concerne alors un système de distribution de gaz thérapeutique, en particulier d'oxygène, tel que défini dans la revendication 1 et ses revendications dépendantes.

Selon le mode de réalisation considéré, le système de distribution de gaz, en particulier d'oxygène médical, de l'invention peut comprendre l'une ou plusieurs des caractéristiques suivantes :
- il comprend un conduit flexible venant se raccorder fluidiquement, via une extrémité amont, au conduit de fourniture de gaz.
- ladite unité de traitement de signal est configurée pour traiter ledit au moins un signal de position de sélecteur et en déduire le débit de gaz correspondant à la position du sélecteur de débit déterminée par le capteur de position.
- ladite unité de traitement de signal est configurée pour traiter ledit au moins un signal de de pression fourni par le capteur de pression de dérivation et en déduire la pression relative régnant dans le conduit de dérivation.
- le microprocesseur de l'unité de traitement de signal est configuré pour analyser ledit au moins un signal de mesure de pression fourni par le capteur de pression de dérivation pour déterminer une obstruction permanente ou temporaire du conduit de dérivation.
- le microprocesseur de l'unité de traitement de signal est configuré pour analyser ledit au moins un signal de position de sélecteur fourni par le capteur de position et ledit au moins un signal de mesure de pression fourni par le capteur de pression de dérivation pour déterminer, i.e. en déduire, un débranchement accidentel de l'extrémité amont du tuyau flexible.
- il comprend en outre des moyens d'alerte configurés pour alerter l'utilisateur en cas de détection d'un défaut de fourniture d'oxygène au patient résultant d'une déconnection ou d'une obstruction du conduit de gaz.
- les moyens d'alerte sont configurés pour délivrer un signal d'alerte audible (i.e. sonore) ou visuel, ou les deux.
- les moyens d'alerte peuvent comprendre un haut-parleur et/ou un avertisseur lumineux, tel qu'une ou des LEDs ou analogues, qui peuvent être commandées pour s'allumer en continu ou de manière intermittente, i.e. clignotement.
- le dispositif de contrôle de débit comprend une plaque à orifices traversée par plusieurs orifices calibrés ayant des tailles différentes correspondant à différents débits de gaz.
- la plaque à orifices a une forme de disque.
- les orifices calibrés ayant des tailles différentes correspondant à différents débits de gaz compris entre 0.1 et 15 L/min.
- le sélecteur de débit coopère avec la plaque à orifices pour sélectionner l'un des orifices calibrés correspondant au débit de gaz désiré.
- le sélecteur de débit comprend un bouton rotatif.
- il comprend en outre un capteur de position agencé pour déterminer la position du sélecteur de débit et configuré pour communiquer au moins un signal de position de sélecteur à une unité de traitement de signal.
- l'unité de traitement de signal est configurée pour traiter le signal de position de sélecteur et en déduire le débit de gaz correspondant à la position du sélecteur de débit déterminée par le capteur de position.
- le conduit de dérivation comprend en outre un capteur de pression de dérivation agencé de manière à déterminer la pression dans ledit conduit de dérivation et configuré pour transmettre au moins un signal de pression de dérivation à ladite unité de traitement de signal.
- la soupape d'échappement comprend une membrane coopérant avec un élément élastique venant exercer une pression sur la membrane de manière à normalement la repousser contre un siège de soupape et ainsi assurer une étanchéité fluidique entre la membrane et le siège de soupape, empêchant tout échappement de gaz vers l'atmosphère ambiante.
- l'élément élastique est un ressort.
- il comprend en outre une source de gaz reliée fluidiquement au dispositif de contrôle de débit par un conduit d'alimentation en gaz.
- la source de gaz comprend une bouteille de gaz sous pression, de préférence d'oxygène de qualité médicale conditionné à une pression de 200 bar abs ou plus.
- il comprend en outre un conduit flexible venant se raccorder fluidiquement, en amont, au conduit de fourniture de gaz et, en aval, à une interface respiratoire délivrant le gaz à un patient.
- il comprend en outre un capteur de pression agencé pour mesurer la pression de gaz au sein de la source de gaz et configuré pour fournir au moins un signal de mesure de pression à l'unité de traitement de signal.
- la soupape d'échappement est configurée pour autoriser un échappement du gaz vers l'atmosphère par décollement de la membrane du siège de soupape, lorsque la pression de dérivation au sein du conduit de pression de dérivation excède une valeur de 250 mbar.
- l'unité de traitement de signal comprend un microprocesseur, de préférence un microcontrôleur.

L'invention va maintenant être mieux comprise grâce à la description détaillée suivante, faite à titre illustratif mais non limitatif, en référence aux figures annexées parmi lesquelles :
Fig. 1 représente un système de distribution d'oxygène médical,
Fig. 2 représente une plaque à orifices calibrés d'un dispositif de contrôle de débit du système de distribution de Fig. 1,
Fig. 3 représente un mode de réalisation d'un système de distribution de gaz thérapeutique amélioré selon l'invention en fonctionnement normal,
Fig. 4 représente le système de la Fig. 4 en cas d'obstruction du tuyau flexible,
Fig. 5 représente le système de la Fig. 4 en cas de débranchement du tuyau flexible.

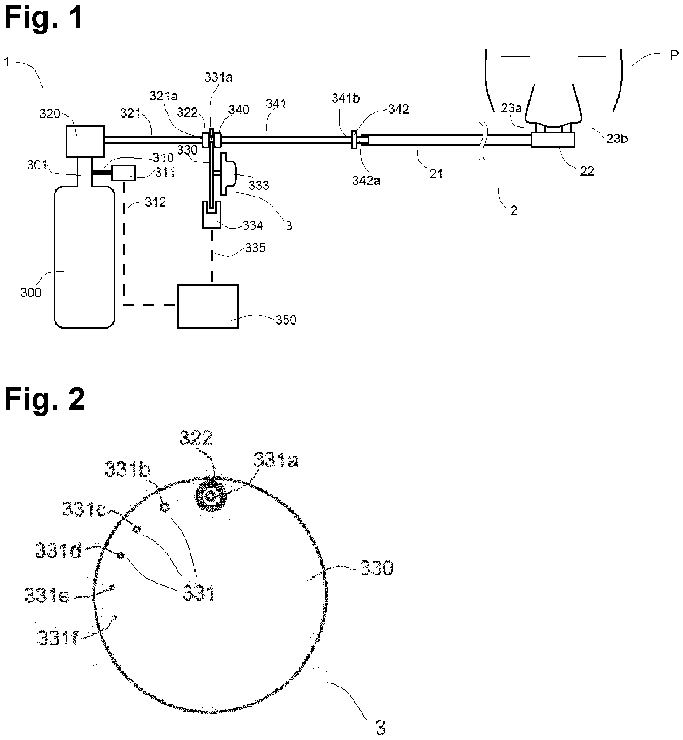

Fig. 1 représente un mode de réalisation d'un système de distribution 1 de gaz thérapeutique, typiquement d'oxygène médical, provenant d'une bouteille d'oxygène sous pression 300 où de l'oxygène de qualité médicale est conditionné à une pression pouvant atteindre 200 bar abs, voire plus.

Un capteur de pression haute 311 est relié fluidiquement, via un port de mesure 310, à un conduit à haute pression 301 en communication fluidique avec le volume interne de la bouteille d'oxygène 300 de manière à y mesurer la pression de l'oxygène gazeuse présent dans le lumen dudit conduit à haute pression 301. Le capteur de pression haute 311 transmet ses signaux de mesure à une unité de traitement de signal 350, via une liaison électrique 312. L'unité de traitement de signal 350 comprend au moins un microprocesseur, tel un microcontrôleur, de préférence agencé sur une carte électronique. Le microprocesseur met en oeuvre un (ou des) algorithmes de traitement des données, i.e. des signaux de mesure ou autres.

Le conduit à haute pression 301 fournit l'oxygène sous pression, aussi appelé oxygène « HP » (HP pour Haute Pression), à un détendeur de gaz 320 qui permet de détendre l'oxygène HP, c'est-à-dire de réduire sa pression jusqu'à une pression plus basse, appelé gaz ou oxygène « BP » (BP pour Basse Pression), par exemple de l'ordre de 5 bar abs.

Le flux d'oxygène gazeux BP obtenu après détente est convoyé par un conduit d'alimentation 321 dont l'extrémité aval 321a aboutit au niveau d'un dispositif de contrôle de débit 3 comprenant une plaque à orifices 330, par exemple un disque, traversée par plusieurs orifices calibrés 331 ayant différentes tailles 331a-331f, i.e. diamètres, correspondant à différents débits de gaz, comme illustré sur Fig. 2.

Le débit souhaité, par exemple un débit de 12 L/min, peut être sélectionné par un utilisateur au moyen d'un sélecteur de débit 333 actionnable par l'utilisateur, tel un bouton rotatif mobile en rotation entre plusieurs positions, par exemple jusqu'à 12 positions différentes pouvant être angulairement réparties, correspond chacune à un débit donné, par exemple des débits compris entre 0 et 15 L/min.

Le sélecteur de débit 333 est couplé, via un axe ou similaire, à la plaque 330 de sorte que la mise en rotation du sélecteur de débit 333, i.e. un bouton rotatif, entraine en rotation la plaque 330, ce qui permet de sélectionner l'orifice calibré 331 correspond au débit désiré.

Par exemple, sur Fig. 2, l'orifice calibré 331 choisi est celui de plus grand diamètre 331a, par exemple 15 L/min. Celui-ci vient se positionner au débouché, c'est-à-dire à l'extrémité aval 321a du conduit d'alimentation 321 de sorte que le flux gazeux circulant dans le lumen du conduit d'alimentation 321 puisse passer au travers de l'orifice calibré 331 de diamètre 331a, ce qui fixe son débit à celui correspondant audit diamètre 331a.

En aval du dispositif de contrôle de débit 3, c'est-à-dire après son passage au travers de l'orifice 331 de la plaque à orifices 330, le flux de gaz au débit contrôlé pénètre dans un conduit de fourniture de gaz 341, via son extrémité amont 341a, qui est en communication fluidique avec le conduit d'alimentation 321, via ledit orifice calibré 331 de diamètre 331a. L'étanchéité fluidique est assurée, de part et d'autre, de la plaque à orifices 330 par des joints toriques 322, 340 venant se positionner autour de l'orifice 331, sur chaque face de la plaque à orifices 330. Typiquement, les joints toriques 322, 340 sont pris en sandwich ou similaire entre la plaque à orifices 330 et les extrémités 321a, 341a du conduit d'alimentation 321 et du conduit de fourniture de gaz 341.

Le conduit de fourniture de gaz 341 comprend à son extrémité aval 341b, un orifice de sortie 342 porté par une prise 342a de connexion fluidique reliée fluidiquement, via un tuyau flexible 21 venant s'emboiter dans la prise 342a de connexion fluidique, à une interface patient 2, telle une canule nasale 22.

La canule nasale 22 comprend un corps principal 22 comprenant une chambre interne ou collecteur de gaz, reliée fluidiquement au tuyau flexible 21 et recevant du gaz convoyé par ledit tuyau flexible 21. La chambre interne du corps principal 22 alimente une paire d'embouts nasals 23a, 23b munis d'orifices de distribution de gaz, lesquels viennent s'insérer dans les narines du patient P pour lui fournir du gaz respiratoire, lors de l'utilisation de la canule nasale 2.

Par ailleurs, il est prévu aussi un capteur de position 334 relié électriquement 335 à l'unité de traitement de signal 350, de manière à pouvoir déterminer instantanément la position du sélecteur de débit 333, e.g. un bouton rotatif, et en déduire ainsi le débit de gaz correspondant à la position du sélecteur de débit 333 ainsi déterminée. A partir de la connaissance de ce débit de gaz et par ailleurs de la pression de gaz mesurée par le capteur de pression haute 311, l'unité de traitement de signal 350 peut calculer, i.e. estimer, une autonomie en gaz de la bouteille 300 et l'afficher sur un afficheur, tel un écran digital ou analogue, et/ou de la télétransmettre à l'utilisateur, par exemple à un serveur, à un téléphone intelligent (i.e. smartphone), à une tablette numérique, à un ordinateur, une montre connectée ou autre.

Avec un tel système de distribution 1 de gaz thérapeutique, lorsque le tuyau flexible 21 est accidentellement pincé, coudé, plié ou analogue, engendrant une obstruction de flux, qui peut être partielle ou totale, et/ou permanente ou temporaire, il va se produire alors une surpression dans le conduit de fourniture de gaz 341 et dans la partie amont du tuyau flexible 21, c'est-à-dire en amont du site où se trouve l'obstruction. Cette surpression peut alors engendrer une déconnexion dudit tuyau flexible 21 de la prise 342a de connexion fluidique car un tuyau flexible 21 n'est généralement pas conçu pour résister à des surpressions gazeuses importantes.

Afin de résoudre ce problème, dans le cadre de la présente invention, il est proposé de modifier le système de distribution 1 de gaz thérapeutique de la Fig. 1 pour permettre de détecter un tel débranchement accidentel résultant d'une obstruction du tuyau flexible 21, tel un pliage, un serrage, une formation de coude ou analogue.

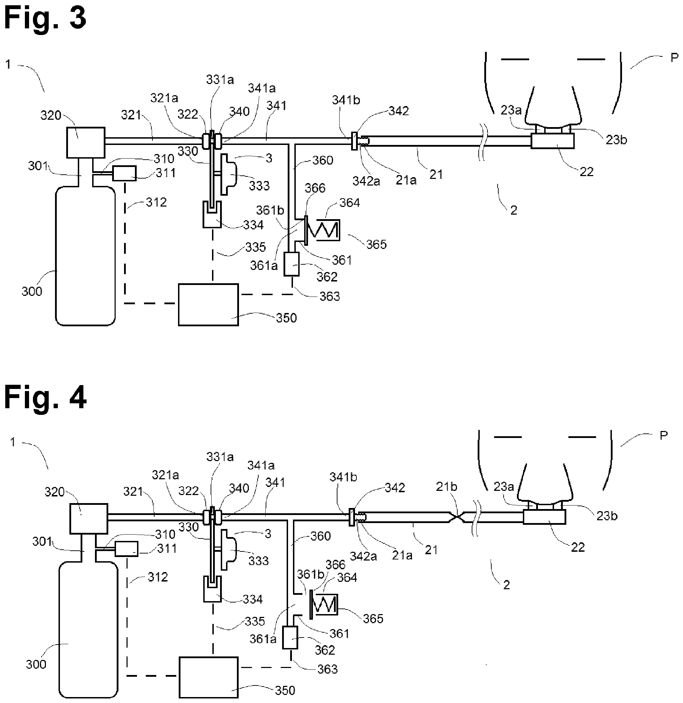

Un mode de réalisation d'un système de distribution 1 de gaz thérapeutique amélioré selon l'invention est montré en Fig. 3.

Plus précisément, afin de résoudre le problème susmentionné, il est proposé d'agencer en aval du dispositif de contrôle de débit 3, un conduit de dérivation 360 venant se raccorder fluidiquement au conduit de fourniture de gaz 341.

Par ailleurs, il est également prévu un second capteur de pression 362 relié fluidiquement audit conduit de dérivation 360 et en liaison électrique avec l'unité de traitement de signal 350, via des câbles électriques ou analogue, ainsi qu'une soupape de décharge 364 en liaison fluidique avec le conduit de dérivation 360, via une chambre intermédiaire 361a agencée entre le conduit de dérivation 360 et la soupape de décharge 364. Cette soupape de décharge 364 permet de contrôler l'évacuation vers l'atmosphère de toute surpression éventuelle apparaissant en cas d'obstruction du tuyau flexible 21, i.e. pincement, coude ou analogue, comme expliqué ci-après.

La soupape de décharge 364 comprend un boitier externe, une membrane 366 coopérant avec un élément élastique 365, tel un ressort, venant exercer une pression sur l'une des faces de la membrane 366 de manière à normalement la repousser contre un siège de soupape 361 et ainsi assurer une étanchéité fluidique entre la membrane 366 et le siège de soupape 361, empêchant alors tout échappement de gaz vers l'atmosphère ambiante, comme visible sur Fig. 3. Le siège de soupape 361 est formé par la région périphérique d'un orifice d'échappement de gaz 361b agencé dans la paroi de la chambre intermédiaire 361a.

Le passage de gaz au travers de l'orifice d'échappement de gaz 361b dépend du niveau de pression s'exerçant dans le conduit de fourniture de gaz 341 et donc aussi dans le conduit de dérivation 360 et la chambre intermédiaire 361a puisqu'ils sont en communication fluidique les uns avec les autres, et par ailleurs de la force de l'élément élastique 365, i.e. typiquement un ressort.

En effet, tant que la pression gazeuse reste inférieure à celle de la force de l'élément élastique 365, le système de distribution 1 de gaz thérapeutique amélioré de l'invention reste dans la configuration montrée en Fig. 3, c'est-à-dire avec la soupape de décharge 364 fermée, i.e. sans décharge de gaz vers l'atmosphère.

Toutefois, en cas d'obstruction 21b du tuyau flexible 21, comme montré en Fig. 4, il apparaît alors une surpression en amont de ladite obstruction, typiquement dans la portion amont du tuyau flexible 21, dans le conduit de fourniture de gaz 341 et par conséquent aussi dans le conduit de dérivation 360 et la chambre intermédiaire 361a.

Lorsque cette surpression excède la force de l'élément élastique 365, il va se produire un décollement de la membrane 366 de son siège 361 et, de ce fait, une libération, i.e. ouverture, de l'orifice d'échappement de gaz 361b étant donné que la membrane 366 va alors être repoussée vers l'arrière, c'est-à-dire vers l'élément élastique 365, par cette surpression gazeuse. L'orifice d'échappement de gaz 361b va alors mettre la chambre intermédiaire 361a, et par conséquent le conduit de dérivation 360 et le conduit de fourniture de gaz 341, en communication fluidique avec l'atmosphère ambiante, ce qui va alors permettre d'échapper une partie du gaz, c'est-à-dire de la surpression.

Avantageusement, la pression d'ouverture de la soupape d'échappement 364, i.e. la force de l'élément élastique 365, est fixée pour être égale à environ deux fois la pression gazeuse pouvant s'exercer dans la chambre intermédiaire 361a, le conduit de dérivation 360 et le conduit de fourniture de gaz 341, en conditions normales d'utilisation, c'est-à-dire en l'absence d'obstruction 21b du tuyau flexible 21, en sélectionnant l'orifice 331 de la plaque à orifices 330 présentant le plus grand diamètre de passage de gaz, donc produisant le flux gazeux le plus grand, et en couplant au système 1, une interface nasale 2 la plus restrictive possible, en termes de flux gazeux distribué, en particulier une canule nasale. Ce choix peut se faire empiriquement via des essais de routine car les niveaux de pression dépendent des caractéristiques des différents dispositifs/éléments utilisés.

A titre d'exemple, pour une canule nasale reliée par un tuyau flexible 21 de 2 mètres de longueur et un orifice 331 de la plaque à orifices 330 de plus grand diamètre de passage de gaz environ égale à 0.75 mm, on peut fixer la pression d'ouverture de la soupape d'échappement 364 à environ 250 mbar.

Toutefois, on peut aussi fixer la pression maximale (i.e. force du ressort) à une valeur « par défaut », typiquement de l'ordre de 500 mbar, car un tel niveau de surpression permet d'assurer une étanchéité fluidique efficace entre le siège 361 et la membrane 366, en conditions normales, indépendamment des autres réglages et des caractéristiques de l'interface respiratoire 2 utilisée. En effet, un tel niveau de pression permet d'éviter toute déconnexion accidentelle du tuyau flexible 21 de la prise 342a de connexion fluidique, tout en assurant un fonctionnement normal du système 1.

Dans le cas d'une obstruction permanente, le second capteur de pression 362, aussi appelé « capteur de pression de dérivation », va mesurer une pression constante de 250 mbar dans la conduite de dérivation 360 et le signal de mesure correspondant est analysé par le microprocesseur de l'unité de traitement de signal 350, qui en déduit qu'il s'agit d'une obstruction permanente car, en fonctionnement normal, la pression 'normale' est toujours inférieure à 250 mbar. Ce défaut représentatif d'une obstruction permanente est affiché sur un afficheur à l'attention de l'utilisateur et/ou fait l'objet du déclenchement d'une alarme sonore et/ou visuelle de manière à informer l'utilisateur et lui permettre d'agir pour remédier au problème, i.e. suppression du défaut.

Dans le cas d'une obstruction temporaire, le capteur de pression de dérivation 362 va la détecter et l'utilisateur est informé de la même manière.

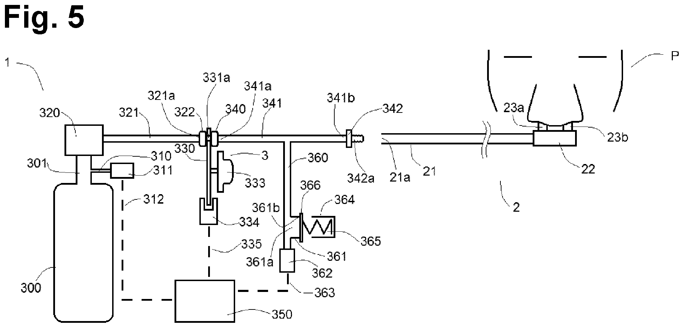

Fig. 5 schématise le cas d'un débranchement accidentel de l'extrémité amont 321a du tuyau flexible 21 de la prise de connexion fluidique 342a du fait, par exemple, d'un mouvement inopiné du patient P. L'orifice de sortie 342 de la prise de connexion fluidique 342a délivre alors le gaz à l'atmosphère.

Dans ce cas, la pression dans le conduit de dérivation 360 est une relation entre le flux gazeux circulant dans la partie aval 341b du conduit de fourniture de gaz 341 et sortant par l'orifice de sortie 342, et la résistance au flux de ces mêmes éléments. Cette pression est normalement inférieure à la force d'ouverture de la soupape de décharge 364, c'est-à-dire inférieure à la pression nécessaire au décollement de la membrane 366 de son siège 361.

Le débit de gaz étant connu grâce au capteur de position 334, comme expliqué ci-avant, l'unité de traitement de signal 350 peut alors calculer, au moyen d'une table de conversion mémorisée par une mémoire de l'unité 350, la perte de pression générée dans la partie aval 341b du conduit de fourniture de gaz 341 par rapport au flux réel qui y circule.

Lorsque le tuyau flexible est correctement raccordé à la prise de connexion fluidique 342a, la pression mesurée dans le conduit de dérivation 360 par le capteur de pression de dérivation 362 est très supérieure à la perte de pression générée dans la partie aval 341b du conduit de fourniture de gaz 341 et au travers de l'orifice de sortie 342.

Par contre, lorsque la pression mesurée dans le conduit de dérivation 360 devient du même ordre que la perte de charge susmentionnée, alors le microprocesseur de l'unité de traitement de signal 350 en déduit qu'un débranchement accidentel de l'extrémité amont 321a du tuyau flexible 21 s'est produit et en informe l'utilisateur, comme précédemment, afin de lui permettre de procéder à une reconnexion fluidique du tuyau flexible 21 à la prise 342a.

En cas de détection d'un défaut de fourniture d'oxygène au patient résultant d'une déconnection ou d'une obstruction du conduit de gaz, il est prévu des moyens d'alerte (non montrés) configurés pour alerter l'utilisateur en délivrant un signal d'alerte audible, c'est-à-dire un signal sonore, par exemple via un haut-parleur, et/ou un signal visuel, en particulier un signal lumineux, par exemple via une ou plusieurs LEDs ou analogues, commandées pour s'allumer de manière continue ou intermittente, c'est-à-dire clignotante.

D'une façon générale, le système de distribution 1 de gaz thérapeutique selon l'invention est particulièrement adapté à une fourniture sécure d'oxygène à un patient pendant un transfert en véhicule d'urgence vers un hôpital ou encore un transfert intra-hospitalier.

## Revendications

1. Système de distribution (1) de gaz thérapeutique comprenant :
- une source de gaz sous pression (300) alimentant un dispositif de contrôle de débit (3) configuré pour délivrer du gaz à différents débits sélectionnables par un utilisateur, ledit dispositif de contrôle de débit (3) comprenant un sélecteur de débit (333),
- un conduit de fourniture de gaz (341) pour convoyer le gaz au débit sélectionné provenant du dispositif de contrôle de débit (3),
- un conduit de dérivation (360) raccordé fluidiquement au conduit de fourniture de gaz (341), et comprenant une soupape d'échappement (364), et
- une unité de traitement de signal (350) à microprocesseur,
**caractérisé en ce que** :
- il comprend en outre un capteur de position (334) agencé pour déterminer la position du sélecteur de débit (333) et configuré pour communiquer au moins un signal de position de sélecteur à l'unité de traitement de signal (350),
- le conduit de dérivation (360) comprend en outre un capteur de pression de dérivation (362) agencé de manière à déterminer la pression dans le conduit de dérivation (360) et configuré pour transmettre au moins un signal de pression de dérivation à l'unité de traitement de signal (350)
- le microprocesseur de l'unité de traitement de signal (350) est configuré pour:
i) déterminer un débit de gaz à partir dudit au moins un signal de position de sélecteur fourni par le capteur de position (334),
ii) calculer, au moyen d'une table de conversion mémorisée par l'unité de traitement de signal (350), une perte de charge générée dans une partie aval (341b) du conduit de fourniture de gaz (341) par rapport audit débit de gaz, et
iii) déterminer qu'un débranchement accidentel de l'extrémité amont (321a) du tuyau flexible (21) s'est produit, lorsque la pression mesurée dans le conduit de dérivation (360) devient du même ordre que la perte de charge calculée.

2. Système selon la revendication précédente, **caractérisé en ce que** le dispositif de contrôle de débit (3) comprend une plaque à orifices (330) traversée par plusieurs orifices calibrés (331) ayant des tailles différentes (331a-331f) correspondant à différents débits de gaz, de préférence la plaque à orifices (330) a une forme de disque.

3. Système selon la revendication 2, **caractérisé en ce que** le sélecteur de débit (333) coopère avec la plaque à orifices (330) pour sélectionner l'un des orifices calibrés (331) correspondant au débit de gaz désiré, de préférence le sélecteur de débit (333) comprend un bouton rotatif.

4. Système selon l'une des revendications précédentes, **caractérisé en ce que** la soupape d'échappement (364) comprend une membrane (366) coopérant avec un élément élastique (365) venant exercer une pression sur la membrane (366) de manière à normalement la repousser contre un siège de soupape (361) et ainsi assurer une étanchéité fluidique entre la membrane (366) et le siège de soupape (361), empêchant tout échappement de gaz vers l'atmosphère ambiante, de préférence l'élément élastique (365) est un ressort.

5. Système selon l'une des revendications précédentes, **caractérisé en ce qu'**il comprend en outre une source de gaz (300) reliée fluidiquement au dispositif de contrôle de débit (3) par un conduit d'alimentation (321) en gaz, de préférence la source de gaz (300) comprend une bouteille de gaz sous pression.

6. Système selon l'une des revendications précédentes, **caractérisé en ce qu'**il comprend en outre un conduit flexible (21) venant se raccorder fluidiquement, en amont, au conduit de fourniture de gaz (341) et, en aval, à une interface respiratoire (22) délivrant le gaz à un patient (P).

7. Système selon l'une des revendications précédentes, **caractérisé en ce qu'**il comprend en outre un capteur de pression (311) agencé pour mesurer la pression de gaz au sein de la source de gaz (300) et configuré pour fournir au moins un signal de mesure de pression à l'unité de traitement de signal (350).

8. Système selon l'une des revendications précédentes, **caractérisé en ce que** la soupape d'échappement (364) est configurée pour autoriser un échappement du gaz vers l'atmosphère par décollement de la membrane (366) du siège de soupape (361), lorsque la pression de dérivation au sein du conduit de pression de dérivation (360) excède une valeur de 250 mbar.

9. Système selon l'une des revendications précédentes, **caractérisé en ce que** l'unité de traitement de signal (350) est configurée pour traiter ledit au moins un signal de pression fourni par le capteur de pression de dérivation (362) et en déduire la pression relative régnant dans le conduit de dérivation (360).

10. Système selon l'une des revendications précédentes, **caractérisé en ce qu'**il comprend des moyens d'alerte configurés pour alerter l'utilisateur en cas de détection d'un défaut de fourniture d'oxygène au patient résultant d'une déconnection ou d'une obstruction du conduit flexible (21).

11. Système selon la revendication 10, **caractérisé en ce que** les moyens d'alerte sont configurés pour délivrer un signal d'alerte audible ou visuel, ou les deux.

## Patentansprüche

1. System zur Abgabe (1) von therapeutischem Gas, das Folgendes beinhaltet:
- eine Quelle von unter Druck stehendem Gas (300), die eine Durchflussregelvorrichtung (3) versorgt, die dazu konfiguriert ist, Gas mit unterschiedlichen, durch einen Benutzer auswählbaren Durchflussraten zu liefern, wobei die Durchflussregelvorrichtung (3) ein Durchflussauswahlmittel (333) beinhaltet,
- eine Gaszufuhrleitung (341), um das Gas, das von der Durchflussregelvorrichtung (3) kommt, mit der ausgewählten Durchflussrate zu transportieren,
- eine Umgehungsleitung (360), die fluidisch mit der Gaszufuhrleitung (341) verbunden ist und ein Auslassventil (364) beinhaltet, und
- eine Signalverarbeitungseinheit (350) mit einem Mikroprozessor, **dadurch gekennzeichnet, dass**:
- es ferner einen Positionssensor (334) beinhaltet, der dazu angeordnet ist, die Position des Durchflussauswahlmittels (333) zu bestimmen, und dazu konfiguriert ist, mindestens ein Auswahlmittelpositionssignal an die Signalverarbeitungseinheit (350) zu kommunizieren,
- die Umgehungsleitung (360) ferner einen Umgehungsdrucksensor (362) beinhaltet, der so angeordnet ist, dass er den Druck in der Umgehungsleitung (360) bestimmen kann, und dazu konfiguriert ist, mindestens ein Umgehungsdrucksignal an die Signalverarbeitungseinheit (350) zu übertragen,
- der Mikroprozessor der Signalverarbeitungseinheit (350) zu Folgendem konfiguriert ist:
i) Bestimmen einer Gasdurchflussrate anhand des mindestens einen Auswahlmittelpositionssignals, das durch den Positionssensor (334) bereitgestellt wird,
ii) Berechnen, mittels einer durch die Signalverarbeitungseinheit (350) gespeicherten Umrechnungstabelle, eines Druckverlusts, der im Verhältnis zur Gasdurchflussrate in einem stromabwärts gelegenen Teil (341b) der Gaszufuhrleitung (341) erzeugt wird, und
iii) Bestimmen, dass eine unbeabsichtigte Trennung des stromaufwärts gelegenen Endes (321a) des flexiblen Schlauchs (21) aufgetreten ist, wenn der in der Umgehungsleitung (360) gemessene Druck die gleiche Größenordnung wie der berechnete Druckverlust erreicht.

2. System nach dem vorhergehenden Anspruch, **dadurch gekennzeichnet, dass** die Durchflussregelvorrichtung (3) eine Lochplatte (330) beinhaltet, die von mehreren kalibrierten Löchern (331) mit unterschiedlichen Größen (331a-331f), die unterschiedlichen Gasdurchflussraten entsprechen, durchquert wird, vorzugsweise die Lochplatte (330) scheibenförmig ist.

3. System nach Anspruch 2, **dadurch gekennzeichnet, dass** das Durchflussauswahlmittel (333) mit der Lochplatte (330) zusammenwirkt, um eine der kalibrierten Öffnungen (331), die der gewünschten Gasdurchflussrate entspricht, auszuwählen, vorzugsweise das Durchflussauswahlmittel (333) einen Drehknopf beinhaltet.

4. System nach einem der vorhergehenden Ansprüche, **dadurch gekennzeichnet, dass** das Auslassventil (364) eine Membran (366) beinhaltet, die mit einem elastischen Element (365) zusammenwirkt, welches einen Druck auf die Membran (366) derart ausübt, dass diese im Normalfall gegen einen Ventilsitz (361) gedrückt wird und so eine Fluiddichtheit zwischen der Membran (366) und dem Ventilsitz (361) gewährleistet, sodass jegliches Austreten von Gas in die Umgebungsatmosphäre verhindert wird, vorzugsweise das elastische Element (365) eine Feder ist.

5. System nach einem der vorhergehenden Ansprüche, **dadurch gekennzeichnet, dass** es ferner eine Gasquelle (300) beinhaltet, die über eine Gasversorgungsleitung (321) fluidisch mit der Durchflussregelvorrichtung (3) verbunden ist, vorzugsweise die Gasquelle (300) eine Druckgasflasche beinhaltet.

6. System nach einem der vorhergehenden Ansprüche, **dadurch gekennzeichnet, dass** es ferner eine flexible Leitung (21) beinhaltet, die stromaufwärts mit der Gaszufuhrleitung (341) und stromabwärts mit einem Atemanschluss (22), der das Gas an einen Patienten (P) liefert, fluidisch verbunden wird.

7. System nach einem der vorhergehenden Ansprüche, **dadurch gekennzeichnet, dass** es ferner einen Drucksensor (311) beinhaltet, der dazu angeordnet ist, den Gasdruck innerhalb der Gasquelle (300) zu messen, und dazu konfiguriert ist, der Signalverarbeitungseinheit (350) mindestens ein Druckmesssignal bereitzustellen.

8. System nach einem der vorhergehenden Ansprüche, **dadurch gekennzeichnet, dass** das Auslassventil (364) dazu konfiguriert ist, ein Austreten des Gases in die Atmosphäre durch Lösen der Membran (366) von dem Ventilsitz (361) zu gestatten, wenn der Umgehungsdruck innerhalb der Umgehungsdruckleitung (360) einen Wert von 250 mbar überschreitet.

9. System nach einem der vorhergehenden Ansprüche, **dadurch gekennzeichnet, dass** die Signalverarbeitungseinheit (350) dazu konfiguriert ist, das mindestens eine Drucksignal, das durch den Umgehungsdrucksensor (362) bereitgestellt wird, zu verarbeiten und daraus den relativen Druck, der in der Umgehungsleitung (360) herrscht, abzuleiten.

10. System nach einem der vorhergehenden Ansprüche, **dadurch gekennzeichnet, dass** es Warnmittel beinhaltet, die dazu konfiguriert sind, den Benutzer zu warnen, wenn ein Fehler bei der Zufuhr von Sauerstoff an den Patienten, der sich aus einer Unterbrechung oder einer Verstopfung der flexiblen Leitung (21) ergibt, detektiert wird.

11. System nach Anspruch 10, **dadurch gekennzeichnet, dass** die Warnmittel dazu konfiguriert sind, ein akustisches oder visuelles Warnsignal oder beides zu liefern.

## Claims

1. System (1) for distributing a therapeutic gas, comprising:
- a source (300) of pressurized gas feeding a flow control device (3) configured to deliver the gas at different flow rates that are selectable by a user, said flow control device (3) comprising a flow rate selector (333),
- a gas supply line (341) for conveying the gas at the selected flow rate from the flow control device (3),
- a bypass line (360) fluidically connected to the gas supply line (341), and comprising an exhaust valve (364), and
- a signal processing unit (350) with microprocessor,
**characterized in that**:
- it further comprises a position sensor (334) arranged so as to determine the position of the flow rate selector (333) and configured to communicate at least one selector position signal to the signal processing unit (350),
- the bypass line (360) further comprises a bypass pressure sensor (362) arranged so as to determine the pressure in the bypass line (360) and configured to transmit at least one bypass pressure signal to the signal processing unit (350),
- the microprocessor of the signal processing unit (350) is configured to:
i) determine a gas flow rate from said at least one selector position signal supplied by the position sensor (334),
ii) compute, by means of a conversion table stored by the signal processing unit (350), a pressure drop generated in a downstream portion (341b) of the gas supply line (341) with respect to said gas flow rate, and
iii) determine that the upstream end (321a) of the flexible tube (21) has been accidentally disconnected, when the pressure measured in the bypass line (360) becomes of the same order as the computed pressure drop.

2. System according to the preceding claim, **characterized in that** the flow control device (3) comprises an orifice plate (330) which is passed through by several calibrated orifices (331) having different sizes (331a-331f) corresponding to different gas flow rates, preferably the orifice plate (330) has the shape of a disc.

3. System according to Claim 2, **characterized in that** the flow rate selector (333) cooperates with the orifice plate (330) so as to select one of the calibrated orifices (331) corresponding to the desired gas flow rate, preferably the flow rate selector (333) comprises a rotary knob.

4. System according to one of the preceding claims, **characterized in that** the exhaust valve (364) comprises a diaphragm (366) cooperating with an elastic element (365) which exerts a pressure on the diaphragm (366) so as to normally push it against a valve seat (361) and thus ensure a fluid-tight seal between the diaphragm (366) and the valve seat (361), preventing any gas from escaping to the ambient atmosphere, preferably the elastic element (365) is a spring.

5. System according to one of the preceding claims, **characterized in that** it further comprises a source (300) of gas fluidically connected to the flow control device (3) by a gas feed line (321), preferably the source (300) of gas comprises a cylinder of pressurized gas.

6. System according to one of the preceding claims, **characterized in that** it further comprises a flexible line (21) which is fluidically connected, upstream, to the gas supply line (341), and, downstream, to a respiratory interface (22) delivering the gas to a patient (P).

7. System according to one of the preceding claims, **characterized in that** it further comprises a pressure sensor (311) arranged so as to measure the gas pressure within the source (300) of gas and configured to supply at least one pressure measurement signal to the signal processing unit (350).

8. System according to one of the preceding claims, **characterized in that** the exhaust valve (364) is configured to allow gas to escape to the atmosphere by detachment of the diaphragm (366) from the valve seat (361), when the bypass pressure within the bypass pressure line (360) exceeds a value of 250 mbar.

9. System according to one of the preceding claims, **characterized in that** the signal processing unit (350) is configured to process said at least one pressure signal supplied by the bypass pressure sensor (360) and to deduce therefrom the relative pressure prevailing in the bypass line (360).

10. System according to one of the preceding claims, **characterized in that** it comprises warning means configured to warn the user in the event of detection of a fault in the supply of oxygen to the patient resulting from a disconnection or an obstruction of the flexible line (21).

11. System according to Claim 10, **characterized in that** the warning means are configured to deliver an audible or visual warning signal, or both.
